# EUROPEAN PATENT APPLICATION

(11) **EP 4 353 153 A1**
(43) Date of publication of application: **17.04.2024**
(21) Application number: 22201507.5
(22) Date of filing: 14.10.2022
(51) Int. Cl.: A61B 5/256, G01L 5/04, G01L 5/06, A61B 5/00

(54) **SYSTEM AND METHOD FOR POSITIONING A SENSOR PATCH ON A USER**

(71) Applicant: Koninklijke Philips N.V., 5656 AG Eindhoven (NL)
(72) Inventor: TE VELDE, Mart, Eindhoven (NL); SMOOR, Divera Lucretia, Eindhoven (NL)
(74) Representative: Philips Intellectual Property & Standards

(57) **Abstract**

A system (10) for positioning a sensor patch on a user is provided, the system comprising a band (20) for creating a pressing force to the sensor patch when the band is stretched, the band comprising a stretchable part (22), and a first marker (25) located on the stretchable part, and a non-stretchable element (30), having a second marker (35), wherein a relative position of the first and second marker indicates an amount of stretch of the stretchable part. The user guided by the relative position of the first (25) and second (35) markers may take actions for the stretchable part (22) of the band to reach a desired stretch, which corresponds to a desired pressing force to the sensor patch. A corresponding method (100) for positioning a sensor patch on a user is also provided.

## Description

### FIELD OF THE INVENTION

The invention relates to a system and a method for positioning a sensor patch on a user. More specifically, it relates to a system comprising a band, at least permitting the monitoring of the stretch applied by the band to the user.

### BACKGROUND OF THE INVENTION

Sensor patches comprising electrodes can be used for electrophysiological measurements, which involve measurements of voltage changes or electric current in biological tissues. These measurements can be used for monitoring biometric parameters of a subject or for diagnostic purposes, e.g., based on electrocardiography (ECG) or electroencephalography (EEG). Another example is for pregnancy monitoring based on electrical-cardiotocography (eCTG), e.g. determining fetal heart rate (FHR), maternal heart rate (MHR) and uterine activity (UA). Sensor patches may comprise wet, dry or semi dry electrodes. For sensor patches which are designed to be re-used, dry electrodes are preferred, as they can be cleaned and they do not require gels or adhesives, which may be depleted after one or more uses. Sensor patches having dry electrodes can be in direct contact with the skin or can be worn on top of the clothes worn by a user.

To ensure good contact of the electrodes with the user, a certain amount of pressing force is required to be applied to the sensor patch and a stretchable band that is worn by the user can be used to create such a pressing force. The pressing force that the band exerts to the sensor patch can be considered proportional to the stretch of the band.

It is important to know if the amount of pressing force is the desired one. In case the pressing force is too high, it may create discomfort to the user. If on the other hand the pressing force is too low, it may lead to a poor contact between the electrode and the skin/clothing contact, resulting to incorrect functioning of the sensor patch and unreliable measurements.

There is thus a need for an improved system comprising a band, being able to at least permit the monitoring of stretch of the band, which indicates an amount of pressing force applied to the sensor patch.

### SUMMARY OF THE INVENTION

It is, inter alia, an object of the invention to provide a system comprising a band and a corresponding method, for positioning a sensor patch on a user, at least permitting the monitoring of the stretch applied by the band to the user. Using the system according to the invention, the user is able to at least observe if the amount of stretch is a desired one, if the band is under-stretched or overstretched and according to certain embodiments to adjust the amount of stretch.

The invention is defined by the independent claims. Advantageous embodiments are defined in the dependent claims.

A first aspect of the invention provides a system for positioning a sensor patch on a user, the system comprising a band for creating a pressing force to the sensor patch when the band is stretched, the band comprising a stretchable part, and a first marker located on the stretchable part, and a non-stretchable element, having a second marker, wherein a relative position of the first and second marker indicates an amount of stretch of the stretchable part.

An advantage thereof is that the user by visual inspection of the system may observe whether the stretchable part of the band has a desired stretch. The desired stretch corresponds to the desired force, i.e., the force desired by design which is required to press the sensor patch against the user. If the amount of stretch is the desired one, then a desired pressing force is achieved, reducing discomfort of the user and ensuring proper contact of the electrode with the skin or the clothes of the user, so that the sensor patch may take electrophysiological measurements according to its specifications. If it is observed that the stretch is less or more than desired, the user or another person can take action to bring the stretch of the band to a desired amount, e.g. by changing to another band which has dimensions more suitable to the size of the user, or by adjusting the amount of stretch, e.g., by using a fastener.

In case that the sensor patch is separable from the band and is designed such that the band can slide over the sensor patch when the user changes a position or moves, an advantage of ensuring that the band is not overstretched, is that motion artifacts may be reduced. If the stretch of the band exceeds a desired amount, then the high pressing force may effectively fasten the sensor patch on the band. As a result, the sliding of the band over the sensor patch will be hindered, leading to the dislocation of the sensor patch with respect to the skin of the user and to the creation of motion artifacts.

In an embodiment, the non-stretchable element may be a ribbon or a bar mounted on the band, the non-stretchable element even when taut, permitting the stretching of the band. An advantage thereof is that when the band is worn, the non-stretchable element does not restrict the stretching of the band, even in the occasion that the band is stretched more than the desired amount. Otherwise, the user could potentially tear and irreparably damage the band, e.g. while attempting to wear it or attempting to increase the stretch more than the desired amount. Thus, these features enable a robust system, being less likely to be accidentally damaged by improper use.

In an embodiment, the stretchable part may comprise at least two first markers, which are symmetrical about an axis extending along a width of the band and the non-stretchable element is mounted on the band, wherein the non-stretchable element comprises at least two second markers, which are symmetrical about the same axis. An advantage thereof is that by having two or more first and second markers, the user is provided with extra markers to check the stretch of the band, resulting in improved usability of the system. For example, if one pair of first and second markers is out of sight of the user e.g., an obstacle is present or the body part where the band is worn may not permit easy inspection, at least another pair of first and second markers may be visible.

According to an embodiment, the non-stretchable element of the system may be a bar, the system comprising one connector, connecting the band with the bar. An advantage of this embodiment is that using a bar connected with only one connector to the band, simplifies the implementation of the system and reduces manufacturing complexity and costs. An advantage of using a bar instead of a ribbon is that the bar does not bend due to gravity, nor changes its shape when it is not taut and thus it is less prone to be tangled with the belt when the band is not worn. Therefore, ease of use of the system is improved.

According to an embodiment, the system may comprise a fastener for adjusting an amount of stretch of the stretchable part. An advantage thereof is that the user is able to adjust the stretch of the band, in order to reach a desired stretch and thus a desired pressing force. In case the system does not comprise a fastener, the user is limited to observing the amount of stretch of the stretchable part of the band.

According to an embodiment, the band comprises a non-stretchable part, wherein the non-stretchable part is less stretchable than the stretchable part, and wherein at least one of the non-stretchable element and the fastener are mounted to the non-stretchable part. The material of the non-stretchable part, being more rigid, permits a more stable and more robust connection with the non-stretchable element and/or the fastener, compared to a similar connection with the stretchable part.

According to an embodiment, the non-stretchable element of the system may be the sensor patch, wherein the second marker is an edge of the sensor patch. The sensor patch itself can also be used for the indication of the amount of stretch of the band. In case that the sensor patch is the only non-stretchable element of the system, the advantage of this embodiment is that the components, the complexity and the manufacturing costs of the system are reduced, as no extra non-stretchable elements are needed. If the sensor patch is used as an additional non-stretchable element, an advantage thereof is that it provides further second markers to the user. Thus, the usability of the system is improved, i.e. the user has the option of consulting at least an extra pair of first and second markers.

In a second aspect of the invention a method for positioning a sensor patch on a user is provided, the method comprising positioning the sensor part on the user, positioning on the user a system as described herein, and monitoring the amount of stretch of the stretchable part of the band indicated by the relative position of the first and second marker.

Additionally, in case the described system comprises a fastener, the method further enables adjusting the amount of stretch of the stretchable part of the band, by securing the fastener in a desired position on the band.

The advantages and technical effects as already described for the system also apply to the corresponding method.

These and other aspects of the invention will be apparent from and elucidated with reference to the embodiments described hereinafter.

### BRIEF DESCRIPTION OF THE DRAWINGS

Figs. 1a - Id show an example of the system for positioning a sensor patch on a user according to the invention.
Figs. 2a - 2d show another example of the system for positioning a sensor patch on a user according to the invention.
Figs. 3a - 3c show another example of the system for positioning a sensor patch on a user according to the invention.
Figs. 4a - 4c show another example of the system for positioning a sensor patch on a user according to the invention.
Figs. 5a - 5c show another example of the system for positioning a sensor patch on a user according to the invention.
Figs. 6a - 6b show an example of the system for positioning a sensor patch on a user according to the invention.
Fig. 7 shows a flowchart of a method for positioning a sensor patch on a user according to the invention.

### DETAILED DESCRIPTION OF EMBODIMENTS

The invention will be described with reference to the Figures. The detailed description and specific examples, while indicating exemplary embodiments of the invention, are intended for purposes of illustration only and are not intended to limit the scope of the invention. These and other features, aspects, and advantages of the present invention will become better understood from the following description, appended claims, and accompanying drawings. The Figures are merely schematic and are not drawn to scale. It should also be understood that the same reference numerals are used throughout the Figures to indicate the same or similar parts.

The invention provides a system and a method for positioning a sensor patch on a user. The sensor patch may be used for electrophysiological measurements e.g., based on electrocardiography (ECG), electroencephalography (EEG) or electrical-cardiotocography (eCTG). The user's body part where the sensor patch should be positioned depends on the measurements to be taken. For example, a sensor patch monitoring ECG should preferably be placed at a chest area of the user, a sensor patch monitoring EEG should preferably be placed at the head of the user, and a sensor patch used for pregnancy monitoring should be placed at the belly of the user.

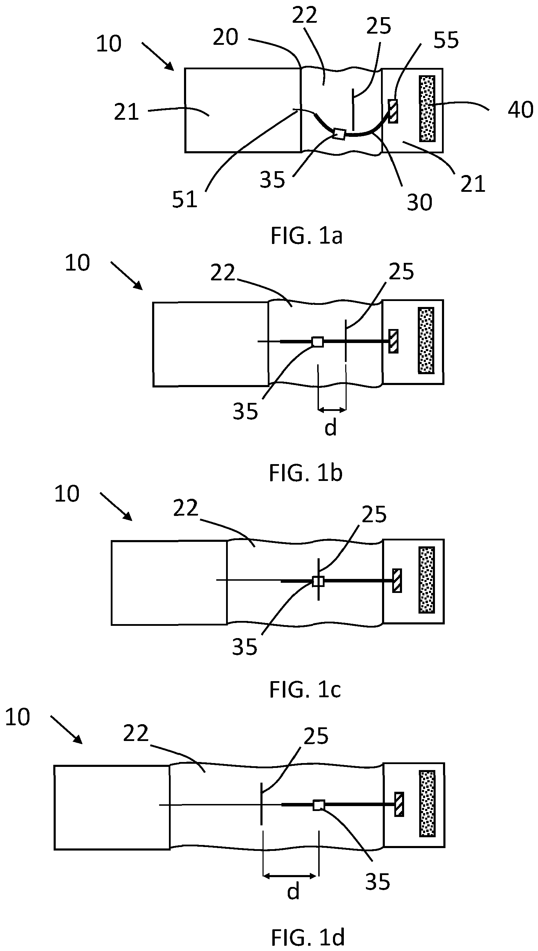

Figs 1a to Id show a system 10 for positioning a sensor patch on a user. The system 10 comprises a band 20, the band having a stretchable part 22 wherein a first marker 25 is located. The system also comprises a non-stretchable element 30, having a second marker 35. In Figs 1a to Id the non-stretchable element 30 is a ribbon, although other options are also available such as a bar. The non-stretchable element's 30 length does not substantially change under tensile forces, e.g. it increases less than 5%, preferably less than 2% and more preferably less than 1% of the length it has in untaut position.

The band 20, e.g., a belt, creates a pressing force to the sensor patch when the band 20 is stretched, thus, keeping the sensor patch positioned on the user. The sensor patch may comprise wet, semi-dry or dry electrodes.

The stretchable part 22 of the band 20 may be made of any suitable materials, e.g. stretch fabrics, having stretchable properties, e.g. nylon, elastane, neoprene. Preferably, the stretchable part 22 of the band 20 is made of materials appropriate for clothing and/or is washable. The stretchable part 22 may stretch in one direction only, i.e., either lengthwise or crosswise or in two directions, i.e., both lengthwise and crosswise. Preferably the stretchable part 22 at least partly recovers to its original position when the band 20 is not stretched. The stretchable material may be able to stretch more than 20%, preferably more than 40% and even more preferably in a range between 30% to 60% of its width and/or length in unstretched position, without losing its ability to at least partially recover to its unstretched position.

Each of the disclosed ranges regarding stretchability of the stretchable part 22 can be combined with each of the disclosed ranges regarding non-stretchability of the non-stretchable element 30.

In order to protect the stretchable material from stretching above a point that it may be damaged, the stretchable part 22 may comprise a protecting element, e.g., a ribbon. The protecting element may restrict the stretchable part 22 from stretching more than 100% to 120% of its width and/or length in unstretched position.

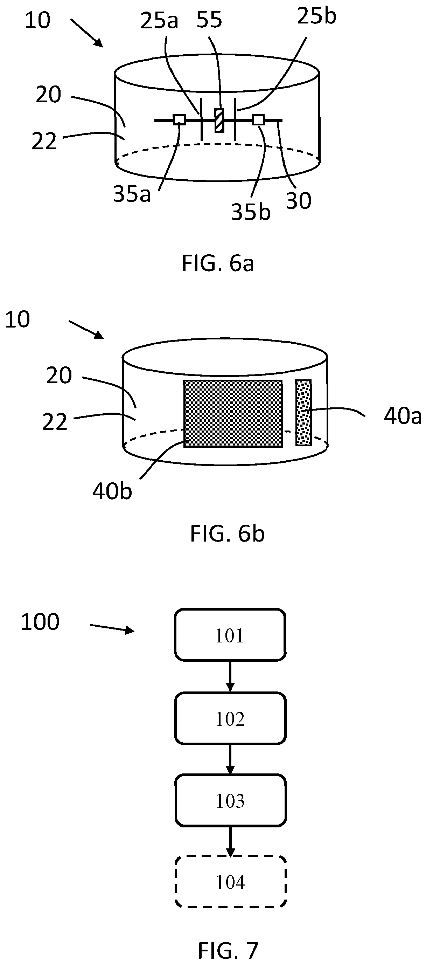

The system 10 as shown in Figs 1a to Id may comprise a fastener 40, for fastening the band around a part of the user's body, e.g., the fastener may be a buckle, a button, a hook-and-loop fastener or any other known means. A fastener is, however, not essential for the invention to work, as is further explained in Fig. 6a.

Figs 1a to Id, show that the system 10 may also comprise a non-stretchable part 21. The non-stretchable part 21 is less stretchable than the stretchable part 22 of the band 20, e.g., it is able to stretch less than 20%, preferably less than 10% and more preferably less than 5% of its width and/or length in unstretched position.

In Figs 1a to Id, the non-stretchable element 30 is a ribbon, mounted on the non-stretchable part 21 of the band 20 with a connector 55 in one end and an elastic joint 51 to the other end. The elastic joint 51 is made of an elastic material, e.g. rubber, which elongates when taut.

Alternatively, the ribbon may be mounted on the stretchable part 22 of the band 20 in both ends, e.g., with a connector 55 in one end, fastening the ribbon to the stretchable part 22 and an elastic joint 51 to the other end, wherein the elastic joint 51 is also fastened to the stretchable part 22. Other solutions are also possible, wherein one end of the non-stretchable element 30 is connected to the non-stretchable part 21 and the other one is connected to the stretchable part 22.

Fig. 1a shows the system 10 in an untaut position, where no tensile force is exerted to the system.

After a sufficient tensile force is exerted to the system, the ribbon is pulled straight as shown in Fig. 1b. The elastic joint 51, permits stretching of the band 20 even when ribbon is taut, i.e., the stretching of the band is not restricted up to the ribbon's length.

The relative position of the first marker 25 located on the stretchable part 22 of the band 20 and the second marker 35 located on the non-stretchable element 30 indicates an amount of stretch of the stretchable part 22. As the stretchable part 22 of the band 20 stretches, its length increases and the position of the first marker 25 changes position with respect to the second marker 35. In an initial position, e.g. the stretchable part 22 being in a position as shown in Fig. 1b, where the stretchable part is stretched enough that the ribbon is pulled straight, there is a distance d, between the first marker 25 and the second marker 35. As the stretch increases the first marker 25 moves closer to the second marker 35 and distance d between the first marker 25 and the second marker 35 decreases. At a point where the distance d becomes zero, the first 25 and second markers 35 coincide and this is the point where the amount of stretch is a desired one.

The first 25 and second 35 markers are shown in the Figures to have a relatively small size, but this is depicted for illustration purposes only. One of the first 25 and the second 35 marker may have larger dimensions from the other e.g., the second marker 35 may have a greater width than the first marker 25. In this example, the desired amount of stretch is not confined to a certain point where the first and second markers coincide, but to a certain range of stretch. For example, the desired amount of stretch is attained, as the first marker 25 reaches one end of the second marker 35 and as the stretch increases and the first marker 25 continues to move with respect to the second marker 35, the stretch is considered to be desired, up to the point that the first marker reaches another end of the second marker 35.

As the stretch continues, after the first marker 25 has reached the second marker 35, i.e., after the desired amount of stretch has been attained, the distance d between the first 25 and second 35 marker increases again as shown in Fig. Id. In Fig. Id the band 20 is shown in an overstretched position, as it has already exceeded the desired stretch and in Fig. 1b the band 20 is shown in an under-stretched position, as it has not reached the desired stretch.

The first marker 25 and second marker 35 are preconfigured to coincide, to the position where the stretchable part 22 of the band 20 has the desired stretch. The desired stretch corresponds to the desired force, i.e., the force desired by design and is required to press the sensor patch against the user, when the system 10 is worn. In an example, the desired force applied to each electrode of the sensor patch is at least 0.1 N, preferably at least 0.5 N and more preferably at least 1 N. Additionally or alternatively, in case the sensor patch has 13 electrodes, and the desired force applied to each electrode is e.g., 0.5 N, a pressing force of at least 13 x 0.5 = 6.5 N is needed. As not all the pressing force of the belt will be distributed to the electrodes, the desired force applied from the band 20 to the sensor patch should be for example doubled, thus it should at least be 13 N. In one non-limiting example, the desired force is a force fulfilling the following requirements: i. it comprises a vector vertical to the skin of the user, which presses the sensor patch on the user, so that the at least one electrode of the sensor patch is able to take the measurements as intended, ii. the sensor patch remains positioned on the user, even when the user changes position or moves and iii. it does not create a prolonged discomfort to the user.

The system 10 may also be considered to work as a scale. For example, by observing the relative position of the first 25 and second 35 markers, e.g., distance d in Figs 1b and Id, the user can estimate how much extra/less stretch is needed to reach the desired amount of stretch.

First 25 and second 35 markers may be printed, sewn or drawn. Additionally, or alternatively instead of being visually perceived by the user, they may also be markers being perceived by the sense of touch, e.g. being protrusions, or having unique tactile properties. Additionally, or alternatively, the user or a third party may also be informed over the relative position of first and second markers using audible guidance. The latter two exemplary solutions would be especially helpful for visually impaired users.

The system 10 may have two or more first markers 25 on the band 20 and two or more second markers 35 on the non-stretchable element 30. In such an example, a first marker 25 and a corresponding second marker 35 make one pair of first 25 and second 35 markers, and the further first and second markers make further corresponding pairs of first and second markers. A first marker 25 and a corresponding second marker 35 make one pair of first and second markers, if they coincide in the position where the band 20 has a desired amount of stretch.

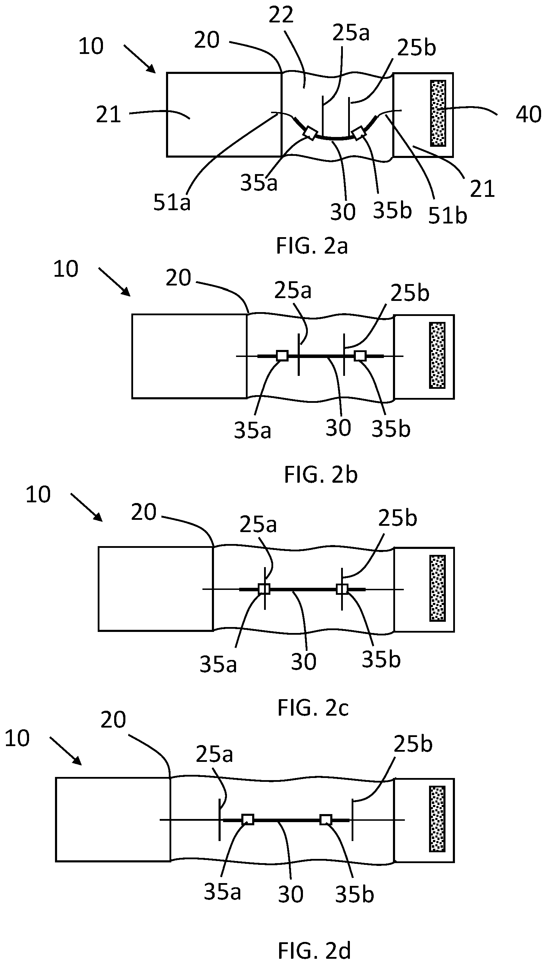

Figs. 2a to 2d show another example of a system 10. Similarly to the system 10 shown in Figs 1a to Id, the system 10 comprises a band 20, having a stretchable part 22 and a non-stretchable part 21. It also comprises a fastener 40. The non-stretchable element 30 is shown to be a ribbon, but it may also be bar. The non-stretchable element 30 in Figs 2a to 2d has two second markers 35a and 35b, while the stretchable part 22 of the band 20 has two first markers 25a and 25b.

Similarly to the example shown in Figs 1a to Id, the non-stretchable element 30 is mounted to the non-stretchable part 21, but it may also be mounted to the stretchable part 22. In Figs 2a to 2d, the non-stretchable element 30 is mounted to the band with elastic joints 51a and 52b to both of its ends.

Fig. 2a shows the system 10 in an untaut position, where no tensile forces are exerted to the system. Fig. 2b shows the system 10 under-stretched but being stretched enough that the ribbon and the elastic joints 51a and 52b are pulled straight. Fig. 2c shows the system 10 where the stretchable part 22 is under a desired stretch, as the first markers 25a and 25b coincide with the second markers 35a and 35b. Fig. 2d shows the system 10 where the stretchable part 22 is overstretched.

In the example shown in Figs 2a to 2d, the first markers 25a and 25b are symmetrical about an axis extending along a width of the band and the second markers 35a and 35b are symmetrical about the same axis. The axis may for example be chosen to pass through the middle, or other points of the non-stretchable element 30. Thus, it is ensured that the first 25 and the second 35 markers are symmetrically aligned. The user may consult the relevant position of either pair of markers, i.e., either first marker 25a compared to second marker 35a, or first marker 25b compared to second marker 35b, to check the stretch of the stretchable part 22.

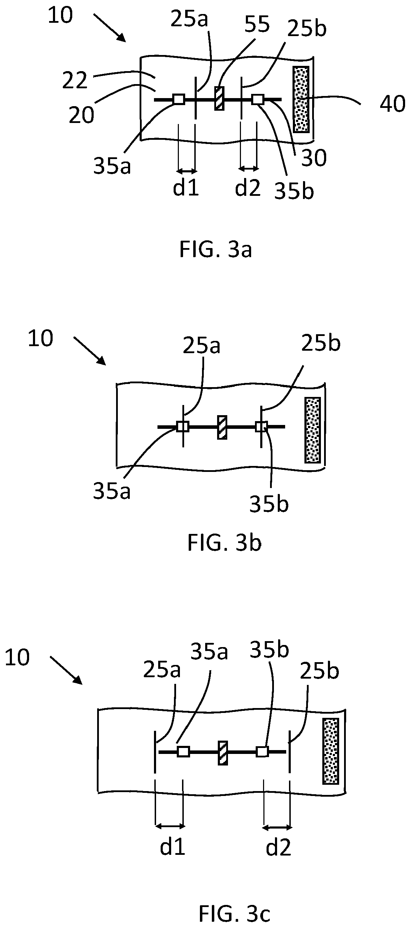

Figs 3a to 3c. provide another embodiment of the invention. In this case, the non-stretchable element 30 is connected to the band 20 at its stretchable part 22 with a single connector 55. The system 10 shown in Figs 3a to 3c, does not comprise a non-stretchable part 21 of the band 20, nevertheless such a feature could also be included. The non-stretchable element 30 is a bar. In this example, there are two first markers 25a and 25b and two second markers 35a and 35b. The connector 55 is located in the middle of the stretchable part 20 and in the middle of bar 30. The first markers 25a and 25b as well as the second markers 35a and 35b have the same distance from the connector 55. Thus, the first markers 25a and 25b and the second markers 35a and 35b are symmetrical with respect to the connector 30.

In further examples only one pair or more pairs of first 25 and second 35 markers can be used. There is no need that the connector 55 is located in the middle of the bar 30, neither is symmetry between pairs of first and second markers, e.g., in the case where the system comprises only one pair of first 25 and second 35 markers.

Starting from the under-stretched position shown in Fig 3a, as the stretchable part 22 of the band 20 stretches, distance d1 of the first marker 25a from the corresponding second marker 35a and distance d2 of the first marker 25b from the corresponding second marker 35b decreases. Thus, the first marker 25a approaches the second marker 35a and the first marker 25b approaches the second marker 35b. When the stretchable part 22 of the band 20 is under the desired stretch, the first markers 25a, 25b coincide with the second markers 35a, 35b, as shown in Fig. 3b. If the stretch of the stretchable part 22 increases more than the desired stretch, then the distance d1 of the first marker 25a with respect to second marker 35a increases and the distance d2 of the first marker 25b with respect with second marker 35b also increases. Thus, the stretchable part 22 has overstretched and the corresponding force to be applied to the sensor patch is greater than desired.

As already explained in the example shown in Figs 2a to 2d, the user may consult either pair of first and second markers, i.e. the relative position of first marker 25a with respect to second marker 35a, and/or the relative position of first marker 25b with respect to second marker 35b. Thus, in case there is an obstacle hindering the user to check one of the pairs, there is the possibility to check the other one.

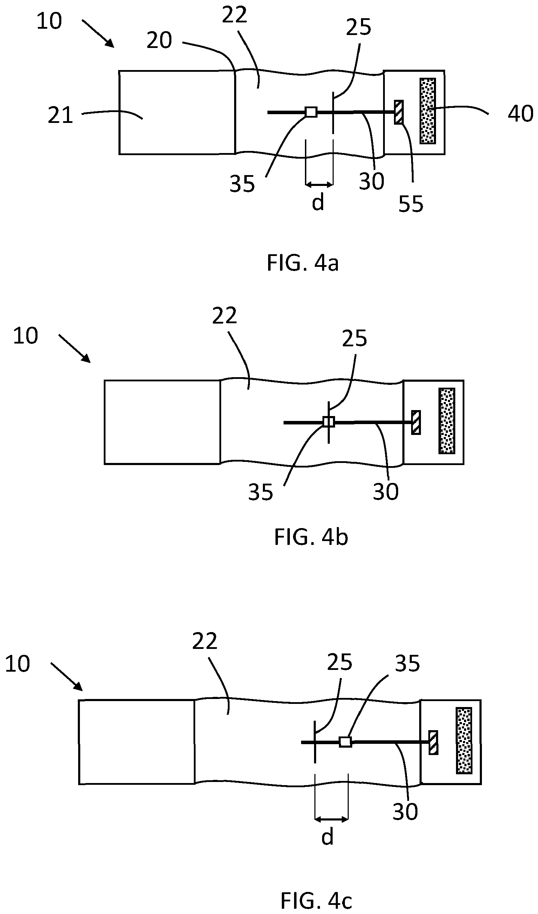

Figs 4a to 4c illustrate another example of implementing the invention. In this example the band 20 comprises a stretchable part 22 and a non-stretchable part 21. It also comprises a fastener 40, for fastening the band 20 around a body part of the user. The non-stretchable 30 is a bar, connected to the non-stretchable part 21 of the band 20. A first marker 25 is located on the stretchable part 22, but more first markers may also be used. A second marker 35 is located on the bar 30, but more second markers may be used as well. Fig. 4a shows the band in an under-stretched position. As the stretch of the stretchable part 22 of the band 20 increases, the distance d of the first marker 25 from the second marker 35 decreases. In the position where the first marker 25 coincides with the second marker 35, as shown in Fig. 4b, the stretchable part 22 has the desired stretch. If the stretch increases farther than the desired stretch, the distance d of the first marker 25 from the second marker 35 increases, as shown in Fig. 4c.

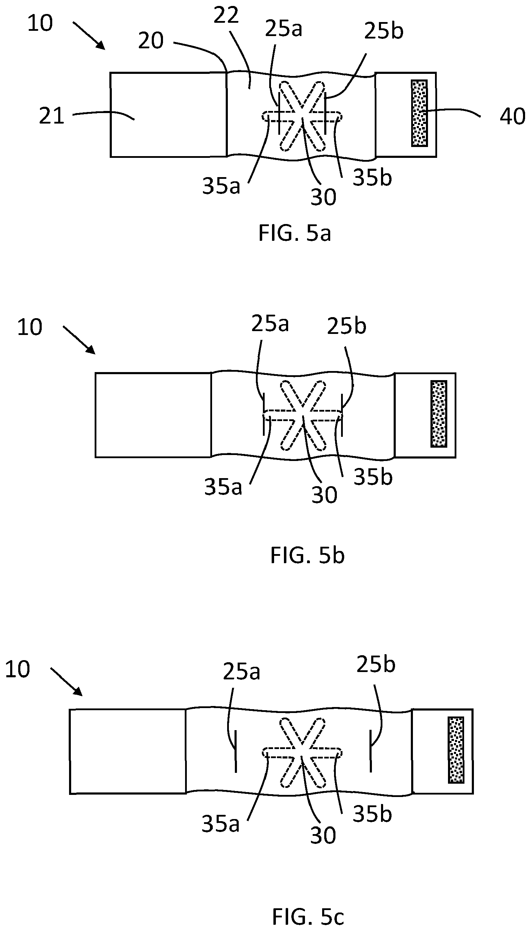

Figs 5a to 5c illustrate another embodiment according to the invention. The system 10 comprises a belt 20. The belt 20 as shown in Figs 5a to 5c comprises a stretchable part 22 and a non-stretchable part 21, but the invention may also work without a non-stretchable part 21. Figs 5a to 5c also show that the belt 20 comprises a fastener 40 but this feature is also optional.

In this embodiment the non-stretchable element 30 is the sensor patch itself. The sensor patch may be separate from the belt 20, alternatively, the sensor patch may be fastened on the belt 20, e.g. using hook-and-loop fasteners, could be sewn on the belt or be fastened with any other known way.

The system 10 as shown in Figs 5a to 5c has two pairs of first and second markers, i.e., a first marker 25a with a corresponding second marker 35a and a first marker 25b with a corresponding second marker 35b, but only one pair or more pairs of first and second markers can also be used. The first markers 25a and 25b are located on the stretchable part 22 of the band 20, and the second markers 35a and 35b are located on the sensor patch.

According to this embodiment the sensor patch is placed on the user and then the band 20 is placed above the sensor patch. Although the sensor patch is at least partially covered by the band 20, the knitting of the band may have such density and/or the transparency of its material should allow the second markers 35a, 35b to be visible. If the second markers are painted on the sensor patch, they should preferably have a high visibility colour. Additionally, or alternatively, the second markers 35a, 35b, may be protrusions on the sensor patch. As a result, they create bumps on the band 20, when it is worn by the user, thus, the second markers 35a, 35b may be perceived either visually, by seeing the bumps, or by the sense of touch. The second markers 35a, 35b may be located in any place of the surface of the sensor patch. In the example shown in Figs 5a to 5c, the second markers are located at edges of the sensor patch.

Fig. 5a shows the system 10 wherein the band 20 is under-stretched and the first markers 25a, 25b, have not reached the respective second markers 35a, 35b. Fig. 5b shows system 10 in a position where the stretchable part 22 has a desired stretch. In this case, first marker 25a coincides with second marker 35a and first marker 25b coincides with second marker 35b. Fig. 5c shows the band 20 in a position that the desired stretch has been exceeded.

Figs 1a to 5c show a band 20 having edges being configured to be fastened together, using a fastener 40. As already indicated a fastener 40 may not be used. Fig. 6a shows such an example, where the band 20 may form a closed loop e.g., may be tubular shaped, without having edges being configured to be fastened together. Such a band may be worn e.g., by pulling it though the legs or head, so that it can be worn around the torso, or around one of the extremities of the human body.

In case the band 20 comprises a fastener 40, the band 20 may have only one size, e.g. a large size. The band 20 is worn around a party of the body of the user, e.g., the belly and using the fastener 40 the amount of stretch can be adjusted. On the other hand, in case the band 20 does not comprise a fastener 40, as shown in Fig. 6a, more than one sizes of the band 20 may be available, depending on the size of the user to wear the band. The user can wear the band 20 and if it is indicated by the system that the amount of stretch is not the desired one, the user may wear another band 20 having different size, e.g., diameter, the size of this another band 20 indicating that its stretch is the desired one. In another example, more than one sizes of a band 20 without a fastener 40 may be needed for the same pregnant user for taking eCTG measurements. As pregnancy progresses, the size of the user's belly increases, thus a larger band 20 without a fastener will be probably needed to maintain a desired amount of stretch of the band holding the sensor patch.

Figs 6a and 6b show the band 20 to only comprise a stretchable part 22, nevertheless, it may also comprise a non-stretchable part. Furthermore, the solution shown in these Figures for indicating a desired stretch of the stretchable part 22 is similar to the one described in Figs 3a to 3c. Nevertheless, solutions described in all Figs 1a to 5c can be implemented with a tubular band, or any other band wherein its edges are not configured to be fastened together.

Fig. 6b shows that a fastener 40 can also be used in case of a tubular band 20, permitting the user to adjust the stretch of the stretchable part 22 of the band 20.

The examples shown in Figs 1a to 6a show a system 10 having one non-stretchable element 30. Nevertheless, more than one non-stretchable elements can be used simultaneously in the same system 10, e.g. one or more bars, one or more ribbons and/or one or more sensor patches.

Fig. 7 shows a flowchart of a method for positioning a sensor patch on a user according to the invention. In step 101, the sensor patch is positioned on the user. The sensor patch may be positioned on any part of the body of the user according to the measurements to be taken, e.g., a sensor patch designed to monitor eCTG should be placed at the belly of the user. In step 102 the user places a system 10 as already described herein on the sensor patch. Thus, the system 10 provides the force which is necessary for the patch to remain positioned on the user, even when the user changes position, or moves. In an example, the sensor patch is configured to be fastened to the system 10, e.g. the sensor patch is configured to be sewn or hooked on the band 20.

After positioning the sensor patch and the system 10 on the user, in step 103, the user and/or a third party is able to monitor the amount of stretch of the stretchable part of the band indicated by the relative position of the first and second marker as already explained herein. In case the system 10 comprises a fastener 40, in an optional step 104, the user and/or a third party, can also adjust the amount of stretch of the stretchable part 22 of the band 20, by securing the fastener 40 in a desired position on the band 20. For example, if the band 20 is under-stretched, the user can increase the stretch of the band 20 up to the point where the relevant position of the first 25 and second 35 markers indicates a desired stretch. Similarly, in case the band 20 is overstretched, the user can decrease the stretch of the band 20 to the point where the relevant position of the first 25 and second 35 markers indicates a desired stretch.

In summary, a system 10 for positioning a sensor patch on a user is provided, the system comprising a band 20 for creating a pressing force to the sensor patch when the band is stretched, the band comprising a stretchable part 22, and a first marker 25 located on the stretchable part, and a non-stretchable element 30, having a second marker 35, wherein a relative position of the first and second marker indicates an amount of stretch of the stretchable part. The user guided by the relative position of the first 25 and second 35 markers may take actions for the stretchable part 22 of the band to reach a desired stretch, which corresponds to a desired pressing force to the sensor patch. A corresponding method 100 for positioning a sensor patch on a user is also provided.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A system (10) for positioning a sensor patch on a user, the system comprising:
a band (20) for creating a pressing force to the sensor patch when the band is stretched, the band comprising a stretchable part (22), and a first marker (25) located on the stretchable part, and
a non-stretchable element (30), having a second marker (35), wherein a relative position of the first and second marker indicates an amount of stretch of the stretchable part.

2. The system of claim 1, wherein:
the non-stretchable element (30) is a ribbon or a bar mounted on the band (20), the non-stretchable element even when taut, permitting the stretching of the band.

3. The system of claims 1 or 2, wherein:
the stretchable part (22) comprises at least two first markers (25a, 25b), which are symmetrical about an axis extending along a width of the band (20), and
the non-stretchable element (30) is mounted on the band, wherein the non-stretchable element comprises at least two second markers (35a, 35b), which are symmetrical about the same axis.

4. The system of claims 1 to 3, wherein the non-stretchable element (30) is a bar, the system comprising one connector (55), connecting the band (20) with the bar.

5. The system of claims 1 to 4, wherein the band (20) comprises a fastener (40) for adjusting an amount of stretch of the stretchable part (22).

6. The system of claims 1 to 5, wherein the band (20) comprises a non-stretchable part (21), wherein the non-stretchable part is less stretchable than the stretchable part (22) and wherein at least one of the non-stretchable element (30) and the fastener (40) are mounted to the non-stretchable part.

7. The system of claims 1 to 6, wherein:
the non-stretchable element (30) is the sensor patch, and
the second marker (35) is an edge of the sensor patch.

8. A method (100) for positioning a sensor patch on a user, the method comprising:
positioning (101) the sensor patch on the user,
positioning (102) on the user a system (10) according to any of claims 1 to 7, and
monitoring (103) the amount of stretch of the stretchable part of the band indicated by the relative position of the first marker (25) and the second marker (35).

9. A method of claim 8, the method comprising:
positioning (102) on the user a system according to claim 5, and
adjusting (104) the amount of stretch of the stretchable part of the band, by securing the fastener in a desired position on the band.
